# EUROPEAN PATENT APPLICATION

(11) **EP 4 290 428 A1**
(43) Date of publication of application: **13.12.2023**
(21) Application number: 22749425.9
(22) Date of filing: 12.01.2022
(51) Int. Cl.: G06N 20/00

(54) **SERVER DEVICE, GENERATION METHOD, ELECTRONIC EQUIPMENT GENERATION METHOD, DATABASE GENERATION METHOD, AND ELECTRONIC EQUIPMENT**

(30) Priority: 03.02.2021 JP 2021015621
(71) Applicant: Sony Group Corporation, Tokyo 108-0075 (JP)
(72) Inventor: TAKATSUKA, Susumu, Tokyo 108-0075 (JP); TETSUKAWA, Hiroki, Tokyo 108-0075 (JP)
(74) Representative: MFG Patentanwälte Meyer-Wildhagen Meggle-Freund Gerhard PartG mbB
(86) International application number: PCT/JP2022/000745
(87) International publication number: WO 2022/168548

(57) **Abstract**

A server device includes: a data acquisition unit that acquires first data regarding a submerged object acquired for a first purpose; and a purpose-specific software generation unit that generates software to be used for a second purpose different from the first purpose on the basis of the first data.

## Description

### TECHNICAL FIELD

The present technology relates to a server device, a generation method, an electronic device generation method, a database generation method, and an electronic device, and particularly relates to a technology of measuring a submerged object.

### BACKGROUND ART

There has been proposed a measurement device that irradiates excitation light having a predetermined wavelength to excite phytoplankton and measures intensity of fluorescence emitted from the excited phytoplankton to measure abundance of the phytoplankton (see, for example, Patent Document 1).

### CITATION LIST

### PATENT DOCUMENT

Patent Document 1: Japanese Patent Application Laid-Open No. 2019-165687

### SUMMARY OF THE INVENTION

### PROBLEMS TO BE SOLVED BY THE INVENTION

In the measurement device described above, dedicated software is installed and executed for each purpose. However, in a case where all the dedicated software is separately generated for each purpose, efficiency of generating the software is reduced.

In view of this, an object of the present technology is to efficiently generate software used for different purposes.

### SOLUTIONS TO PROBLEMS

A server device according to the present technology includes: a data acquisition unit that acquires first data regarding a submerged object acquired for a first purpose; and a purpose-specific software generation unit that generates software to be used for a second purpose different from the first purpose on the basis of the first data.

Therefore, the software to be used for the second purpose can be generated on the basis of the first data acquired for the first purpose different from the second purpose.

In the server device according to the above present technology, the purpose-specific software generation unit may generate software to be used for the first purpose on the basis of second data regarding a submerged object acquired for the second purpose.

Therefore, the software to be used for the first purpose can be generated on the basis of both the first data regarding the submerged object acquired for the first purpose and the second data acquired for the second purpose different from the first purpose.

In the server device according to the above present technology, the purpose-specific software generation unit may generate software to be used for the first purpose on the basis of the first data.

Therefore, both the software to be used for the first purpose and the software to be used for the second purpose can be generated on the basis of the first data acquired for the first purpose.

In the server device according to the above present technology, the purpose-specific software generation unit may generate software to be used for the first purpose and the software to be used for the second purpose on the basis of the first data and second data regarding a submerged object acquired for the second purpose.

Therefore, both the software to be used for the first purpose and the software to be used for the second purpose can be generated on the basis of both the first data acquired for the first purpose and the second data acquired for the second purpose.

The server device according to the above present technology may further include an identification program generation unit that generates an identification program for identifying the submerged object in software to be used for the first purpose and the software to be used for the second purpose.

Therefore, it is possible to identify the submerged object by using the identification program in the software to be used for the first purpose and the software to be used for the second purpose.

In the server device according to the above present technology, the identification program may be used in common to the software to be used for the first purpose and the software to be used for the second purpose.

Therefore, it is possible to identify the submerged object by using the common identification program in the software to be used for the first purpose and the software to be used for the second purpose.

In the server device according to the above present technology, a database used to generate the identification program may be generated on the basis of the first data and second data regarding a submerged object acquired for the second purpose.

Therefore, the database is generated on the basis of both the first data regarding the submerged object acquired for the first purpose and the second data regarding the submerged object acquired for the second purpose.

In the server device according to the above present technology, at least part of a submerged object to be identified may be different between the first purpose and the second purpose.

Therefore, the software to be used for the second purpose can be generated on the basis of the first data acquired for the first purpose in which at least part of the submerged object to be identified is different.

In the server device according to the above present technology, an operation performed when the submerged object to be identified is detected may be different between the first purpose and the second purpose.

Therefore, the software to be used for the second purpose can be generated on the basis of the first data acquired for the first purpose in which the operation performed when the submerged object to be identified is detected is different.

A generation method according to the present technology includes: acquiring first data regarding a submerged object acquired for a first purpose; and generating software to be used for a second purpose different from the first purpose on the basis of the first data.

Such the generation method can also obtain effects similar to those of the server device according to the above present technology.

An electronic device generation method according to the present technology includes: acquiring first data regarding a submerged object acquired for a first purpose; generating software to be used for a second purpose different from the first purpose on the basis of the first data; and storing the software in a medium.

The electronic device generation method according to the above present technology may further include: acquiring second data regarding a submerged object acquired for the second purpose; acquiring an identification result obtained by processing the second data by using the software to be used for the second purpose; and storing the identification result in the medium.

Such the electronic device generation method can also obtain effects similar to those of the server device according to the above present technology.

A database generation method according to the present technology includes: acquiring first data regarding a submerged object acquired for a first purpose; extracting, from the first data, a first data portion necessary for generating software to be used for a second purpose different from the first purpose; and storing all or part of the first data in a medium such that the first data portion is identifiable.

The database generation method according to the above present technology may further include: acquiring second data regarding a submerged object acquired for the second purpose; and integrating the second data and the first data portion and storing the integrated second data and first data portion in a medium as information necessary for generating the software to be used for the second purpose.

Such the database generation method can also obtain effects similar to those of the server device according to the above present technology.

An electronic device according to the present technology includes a medium storing the database.

Such the electronic device can also obtain effects similar to those of the server device according to the above present technology.

### BRIEF DESCRIPTION OF DRAWINGS

Fig. 1 illustrates a configuration of an underwater measurement system according to an embodiment.
Fig. 2 illustrates a usage example of a measurement device.
Fig. 3 illustrates a configuration of a measurement device.
Fig. 4 is a flowchart showing a procedure of measurement operation processing.
Fig. 5 is a flowchart showing a procedure of purpose-specific measurement operation processing.
Fig. 6 illustrates an example of a purpose-specific operation.
Fig. 7 illustrates a configuration of a server device.
Fig. 8 shows a submerged object table.
Fig. 9 shows detailed information of a submerged object table.
Fig. 10 is a flowchart showing a procedure of generation processing.
Fig. 11 illustrates a configuration of a measurement device according to a modification example.

### MODE FOR CARRYING OUT THE INVENTION

Hereinafter, embodiments will be described in the following order.
<1. Configuration of Underwater Measurement System>
<2. Measurement Device>
   [2-1. Usage Example of Measurement Device]
   [2-2. Configuration of Measurement Device]
   [2-3. Measurement Processing by Measurement Device]
   [2-4. Example of Purpose-Specific Operation]
<3. Server Device>
   [3-1. Configuration of Server Device]
   [3-2. Generation Processing]
<4. Another Configuration Example of Underwater Measurement System>
<5. Summary>
<6. Present Technology>

### <1. Configuration of Underwater Measurement System>

First, a configuration of an underwater measurement system 1 according to an embodiment of the present technology will be described.

The underwater measurement system 1 is a system that measures a submerged object such as a microorganism or microplastic existing in water. The measurement here is a concept including at least one of identification of a type or a characteristic of the submerged object and recording or storing of an image captured under the water and, in a broad sense, is a concept including investigation or exploration of the submerged object.

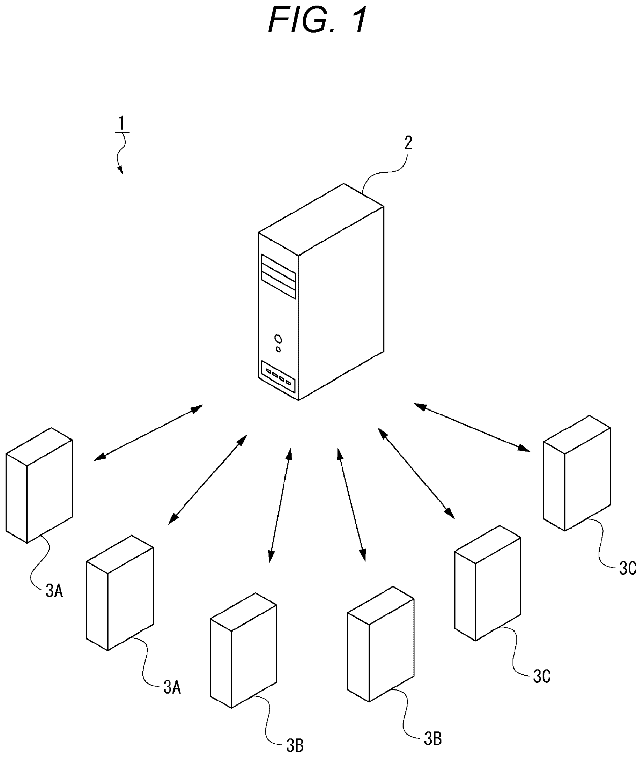

Fig. 1 illustrates a configuration of the underwater measurement system 1. As illustrated in Fig. 1, the underwater measurement system 1 includes a server device 2 and measurement devices 3A, 3B, and 3C. As described later in detail, the measurement devices 3A, 3B, and 3C have different purposes of use and different software to be executed, but have the same configuration. Note that, when the measurement devices 3A, 3B, and 3C are collectively described, the measurement devices will be referred to as a measurement device 3.

The server device 2 can communicate with the measurement device 3, acquires data of a submerged object measured by the measurement device 3, generates software to be executed by the measurement device 3, and transmits the software to the measurement device 3. Note that the generation of the software includes update of the software.

The measurement device 3 executes the software generated by the server device 2 to measure a submerged object. Further, the measurement device 3 performs a specific operation in a case where a target submerged object (hereinafter, referred to as a target object) set in advance for each piece of software is detected.

### <2. Measurement Device>

### [2-1. Usage Example of Measurement Device]

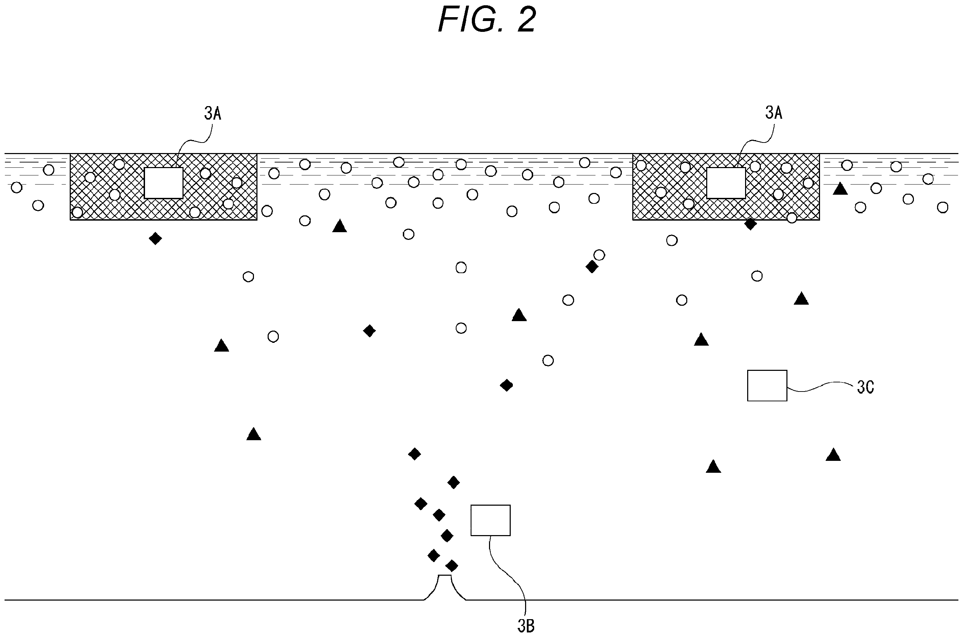

Fig. 2 illustrates a usage example of the measurement device 3. The measurement device 3 is used for various purposes such as marine biological research, aquaculture water quality measurement, fishing spot selection investigation, microplastic measurement, ocean development influence investigation, ship ballast water investigation, marine resource exploration, blue carbon measurement, and global warming investigation.

For example, as illustrated in Fig. 2, the measurement device 3A is arranged in a cage and is used for aquaculture water quality measurement as a first purpose. The measurement device 3B is arranged in deep sea and is used for deep-sea biological research (marine biological research) as a second purpose. The measurement device 3C is arranged in the sea and is used for microplastic measurement as a third purpose. In the present example, three types of measurement devices 3A, 3B, and 3C having different purposes (software) will be described as an example. However, the number of purposes is not limited as long as the measurement devices 3 are used for two or more different purposes (software). Further, the first purpose, the second purpose, and the third purpose are merely examples, and other purposes may be used.

Therefore, software for aquaculture water quality measurement is executed in the measurement device 3A, software for deep-sea biological research is executed in the measurement device 3B, and software for microplastic measurement is executed in the measurement device 3C.

For example, in the software executed by the measurement device 3A, as indicated by circles in Fig. 2, harmful plankton that are harmful in aquaculture are set as the target objects, and the presence or absence of the harmful plankton serving as the target objects, the number of harmful plankton, and the like are detected. Further, in the software executed by the measurement device 3B, as indicated by squares in Fig. 2, deep-sea organisms living in the deep sea are set as the target objects, and the presence or absence of the deep-sea organisms serving as the target objects and the number of deep-sea organisms are detected. Further, in the software executed by the measurement device 3C, as indicated by triangles in Fig. 2, microplastics floating in the sea are set as the target objects, and the presence or absence of the microplastics serving as the target objects and the number of microplastics are detected.

### [2-2. Configuration of Measurement Device]

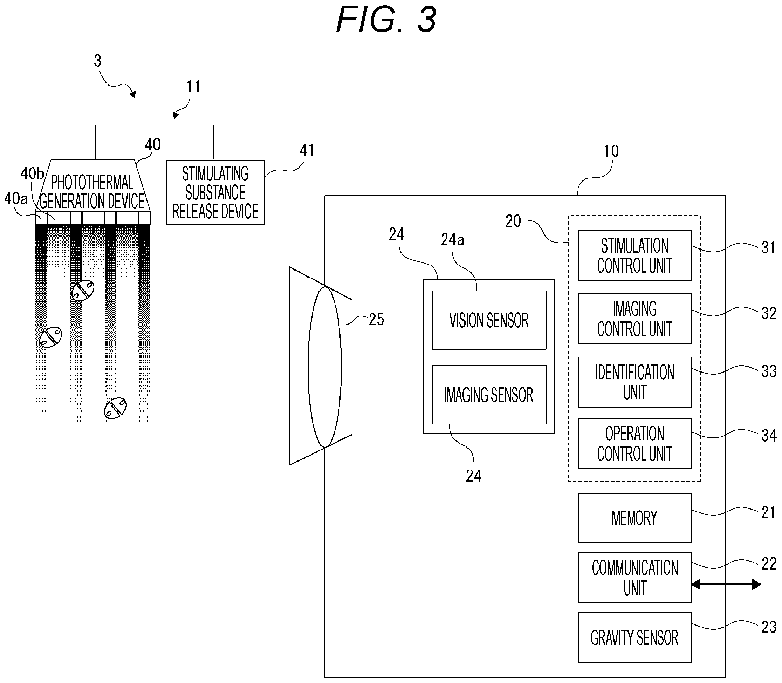

Fig. 3 illustrates a configuration of the measurement device 3. As illustrated in Fig. 3, the measurement device 3 includes a measurement unit 10 and a stimulus generation unit 11.

The measurement unit 10 is a device that can appropriately control the measurement device 3 to measure a target object by using, for example, at least one of taxis or fluorescence of a submerged object (mainly aquatic organism).

Here, the taxis is innate behavior of an organism reacting to a directional external stimulus. Examples of the external stimulus include light, pressure, gravity, a chemical substance (pheromone), electricity, temperature, and contact. Further, for example, taxis with respect to light is referred to as phototaxis, and taxis with respect to a temperature is referred to as thermotaxis. Further, movement in a direction approaching a source of the external stimulus is referred to as positive taxis, and movement in a direction away from the source of the external stimulus is referred to as negative taxis.

For example, a genus of Euglena of a flagellate protozoan moves towards a light source upon exposure to light. In this example, the directional external stimulus is light, and the genus of Euglena exhibits positive phototaxis.

Further, when placed in an environment with a temperature gradient, nematodes move toward a temperature field (approximately 25°C) that is appropriate for the nematodes. In this example, the directional external stimulus is temperature, and the nematodes exhibit the thermotaxis.

As described above, it is known that specific organisms exhibit taxes. Note that organisms exhibiting taxes are found in both plants and animals.

Further, fluorescence is a phenomenon in which, when irradiated with excitation light having a predetermined wavelength, a submerged object is excited to emit light having a wavelength different from that of the excitation light.

The measurement device 3 measures a target object by using at least one of such taxis or fluorescence of the submerged object.

The measurement unit 10 includes a control unit 20, a memory 21, a communication unit 22, a gravity sensor 23, an imaging unit 24, and a lens 25.

The control unit 20 includes, for example, a microcomputer including a central processing unit (CPU), a read only memory (ROM), and a random access memory (RAM) and performs overall control of the measurement device 3. In the present embodiment, the control unit 20 functions as a stimulation control unit 31, an imaging control unit 32, an identification unit 33, and an operation control unit 34. Note that the stimulation control unit 31, the imaging control unit 32, the identification unit 33, and the operation control unit 34 will be described later in detail.

Further, the control unit 20 performs processing of reading data stored in the memory 21, processing of storing data in the memory 21, and transmission and reception of various kinds of data to and from the server device 2 via the communication unit 22.

The memory 21 includes a nonvolatile memory. The communication unit 22 performs wired or wireless data communication with the server device 2. The gravity sensor 23 detects gravitational acceleration (direction of gravity) and outputs a detection result to the control unit 20. Note that the measurement device 3 may not include the gravity sensor 23.

The imaging unit 24 includes a vision sensor 24a and an imaging sensor 24b. The vision sensor 24a is a sensor called dynamic vision sensor (DVS) or event-based vision sensor (EVS). The vision sensor 24a captures an image of a predetermined imaging range through the lens 25.

The vision sensor 24a is an asynchronous image sensor in which a plurality of pixels including photoelectric conversion elements is two-dimensionally arrayed and, in addition, a detection circuit that detects an address event in real time is provided in each pixel. Note that the address event is an event that occurs in each address allocated to each of the plurality of two-dimensionally arrayed pixels and is, for example, an event in which a current value of a current based on a charge generated in the photoelectric conversion element or a change amount thereof exceeds a certain threshold.

The vision sensor 24a detects whether or not the address event occurs for each pixel, and, in a case where the occurrence of the address event is detected, the vision sensor reads a pixel signal as pixel data from the pixel in which the address event has occurred.

The vision sensor 24a performs an operation of reading a pixel signal on the pixel in which the occurrence of the address event has been detected and thus can perform reading at an extremely high speed, as compare with a synchronous image sensor that performs a reading operation on all pixels at a predetermined frame rate, and has a small amount of data read as one frame.

Therefore, the measurement device 3 can detect a motion of the target object more quickly by using the vision sensor 24a. Further, the vision sensor 24a can reduce the amount of data and also reduce power consumption.

The imaging sensor 24b is, for example, a charge coupled device (CCD) or complementary metal-oxide-semiconductor (CMOS) image sensor, and a plurality of pixels including photoelectric conversion elements is two-dimensionally arrayed. The imaging sensor 24b captures an image of a predetermined imaging range through the lens 25 at certain intervals according to a frame rate to generate image data. Note that the measurement unit 10 can use a zone plate, a pinhole plate, or a transparent plate, instead of the lens 25.

The vision sensor 24a and the imaging sensor 24b are arranged to capture an image of substantially the same imaging range through the lens 25. For example, a one-way mirror (not illustrated) is only required to be arranged between the vision sensor 24a and the imaging sensor 24b and the lens 25 such that one part of light dispersed by the one-way mirror is incident on the vision sensor 24a, and the other part thereof is incident on the imaging sensor 24b.

The stimulus generation unit 11 is a device that generates (outputs) an external stimulus within the imaging range captured by the imaging unit 24 to give the external stimulus to an organism existing within the imaging range and includes a photothermal generation device 40 and a stimulating substance release device 41.

The photothermal generation device 40 includes an illumination device 40a (light source) that irradiates the imaging range with light and a heat source device 40b (heat source) that applies heat to the imaging range. The illumination device 40a is driven under the control of the control unit 20 and can change a wavelength and intensity of the light with which the imaging range is irradiated. The heat source device 40b is driven under the control of the control unit 20 and can change a temperature of the imaging range.

The stimulating substance release device 41 includes, for example, a container storing a pheromone (stimulating substance, chemical substance) inside and having an opening/closing door and can release the pheromone toward the imaging range by opening the opening/closing door under the control of the control unit 20.

Note that the stimulus generation unit 11 only needs to include at least one device that generates an external stimulus.

Therefore, unlike a device that measures a wavelength and light intensity of a fluorescence reaction, the measurement device 3 can measure not only phytoplankton but also a wide range of submerged objects including aquatic microorganisms such as phytoplankton, zooplankton, and larvae of aquatic organisms and fine particles in water such as microplastics, dust, sand, and marine snow.

### [2-3. Measurement Operation Processing]

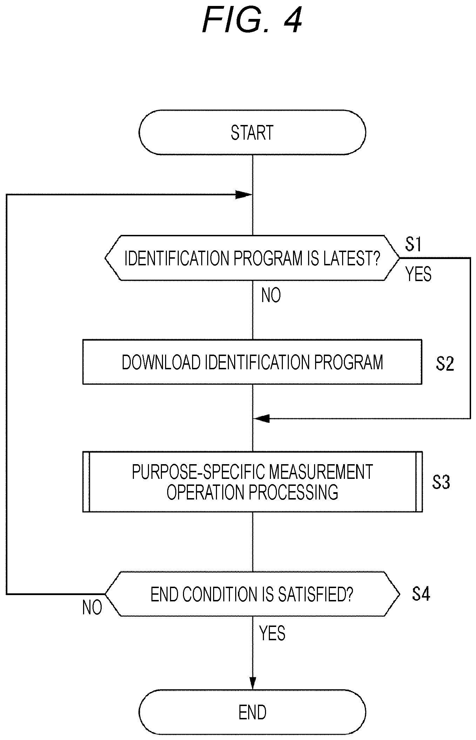

Fig. 4 is a flowchart showing a procedure of measurement operation processing. The control unit 20 executes the measurement operation processing of Fig. 4 by executing purpose-specific software (purpose-specific program) stored in the memory 21.

In step S1, the control unit 20 determines whether or not an identification program stored in the memory 21 is the latest. Here, the control unit 20 determines that a version of the identification program stored in the memory 21 is the latest when the version thereof matches with a version of the identification program provided by the server device 2 and determines that the version is not the latest when the version does not match. Note that the identification program is a program for identifying a submerged object as described later in detail.

Then, in a case where it is determined that the identification program stored in the memory 21 is not the latest (No in step S1), the control unit 20 downloads the latest identification program from the server device 2 and stores the latest identification program in the memory 21 in step S2. Further, in a case where it is determined that the identification program stored in the memory 21 is the latest (Yes in step S1), the processing proceeds to step S3.

In step S3, the control unit 20 executes purpose-specific measurement operation processing of measuring a target object for each purpose or performing an operation when the target object is detected. Note that the purpose-specific measurement operation processing will be described later in detail.

Then, in step S4, the control unit 20 determines whether or not an end condition for ending the measurement operation processing is satisfied, and, in a case where it is determined that the end condition for ending the measurement operation processing is satisfied (Yes in step S4), the measurement operation processing ends. Note that the end condition for ending the measurement operation processing is that, for example, a predetermined time has elapsed, a user instruction for ending the measurement operation processing has been input, or the like.

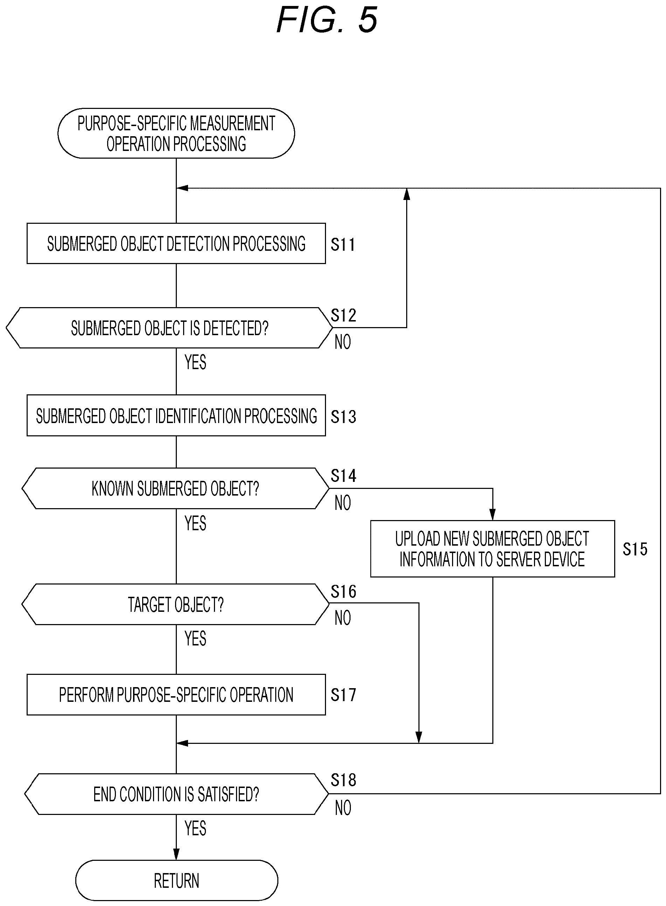

Fig. 5 is a flowchart showing a procedure of the purpose-specific measurement operation processing. As shown in Fig. 5, in step S11, the control unit 20 executes submerged object detection processing of detecting a submerged object.

Here, the stimulation control unit 31 operates the stimulus generation unit 11 so as to generate an external stimulus according to a taxis condition or fluorescence condition of an organism from the stimulus generation unit 11 in accordance with an operation timesheet designated in advance. Further, the imaging control unit 32 controls the imaging unit 24 to capture an image of the imaging range and acquires pixel data and image data.

Then, the identification unit 33 detects a submerged object existing within the imaging range on the basis of the image (pixel data) captured by the vision sensor 24a. For example, the identification unit 33 creates one piece of frame data on the basis of pixel data input within a predetermined period and detects, as a submerged object, a pixel group within a predetermined range in which a motion has been detected in the frame data.

In step S12, the identification unit 33 determines whether or not a submerged object has been detected in step S11 described above. As a result, in a case where it is determined that a submerged object has been detected (Yes in step S11), the identification unit 33 performs submerged object identification processing of identifying the kind of the detected submerged object.

Here, the identification unit 33 first acquires environment information indicating an environment in which the image has been captured. The environment information may include not only a condition of the external stimulus generated from the stimulus generation unit 11 but also the direction of gravity detected by the gravity sensor 23 and external environment information acquired via the communication unit 22. Note that the external environment information can be electrical conductivity, temperature, ph, concentration of gas (e.g. methane, hydrogen, and helium), concentration of metal (e.g. manganese and iron), and the like.

Further, the identification unit 33 derives identification information of the detected submerged object on the basis of the image (pixel data and image data). Here, the identification unit 33 tracks the submerged object between a plurality of pieces of frame data by pattern matching or the like. Then, the identification unit 33 derives a moving direction with respect to a stimulation source, a speed, a trajectory, and the like as the identification information on the basis of a tracking result of the object.

Further, regarding the submerged object from which the identification information has been derived, the identification unit 33 extracts an image portion corresponding to the submerged object from the image (image data) captured by the imaging sensor 24b. Then, the identification unit 33 derives a size of the object, the presence or absence of a tactile sense, and the like as the identification information by image analysis on the basis of the extracted image portion. Note that a known method can be used for the image analysis, and thus description thereof is omitted here.

Thereafter, the identification unit 33 derives a confidence rate between the detected submerged object and a submerged object shown in a submerged object table described later by executing the identification program described later in detail on the basis of the derived environment information and identification information. Note that the confidence rate is a conformability rate indicating whether or not a submerged object is correctly identified.

In step S14, the identification unit 33 determines whether or not the detected submerged object includes a submerged object having the confidence rate of a predetermined first threshold or more, that is, whether or not the detected submerged object is a known submerged object. As a result, in a case where it is determined that the detected submerged object is not a known submerged object (No in step S14), in step S15, the identification unit 33 stores the environment information, the identification information, and the extracted image portion (hereinafter, those will be collectively referred to as submerged object information) as new submerged object information (identification result) in the memory 21 (medium) and transmits (uploads) the new submerged object information to the server device 2.

Meanwhile, in a case where it is determined that the detected submerged object is a known submerged object (Yes in step S14), in step S16, the identification unit 33 determines whether or not the detected submerged object is a target object set in advance for each purpose. Here, in a case where the confidence rate between the detected submerged object and the target object is equal to or more than a second threshold that is higher than the first threshold, it is determined that the detected submerged object is the target object.

As a result, in a case where it is determined that the detected submerged object is the target object (Yes in step S16), the operation control unit 34 performs an operation set in advance for each purpose (hereinafter, referred to as a purpose-specific operation) in step S17.

In step S18, the control unit 20 determines whether or not the end condition for ending the purpose-specific measurement operation processing is satisfied. Then, the control unit 20 repeats steps S11 to S18 until the end condition for ending the purpose-specific measurement operation processing is satisfied and ends the purpose-specific measurement operation processing in a case where the end condition for ending the purpose-specific measurement operation processing is satisfied (Yes in step S18).

### [2-4. Example of Purpose-Specific Operation]

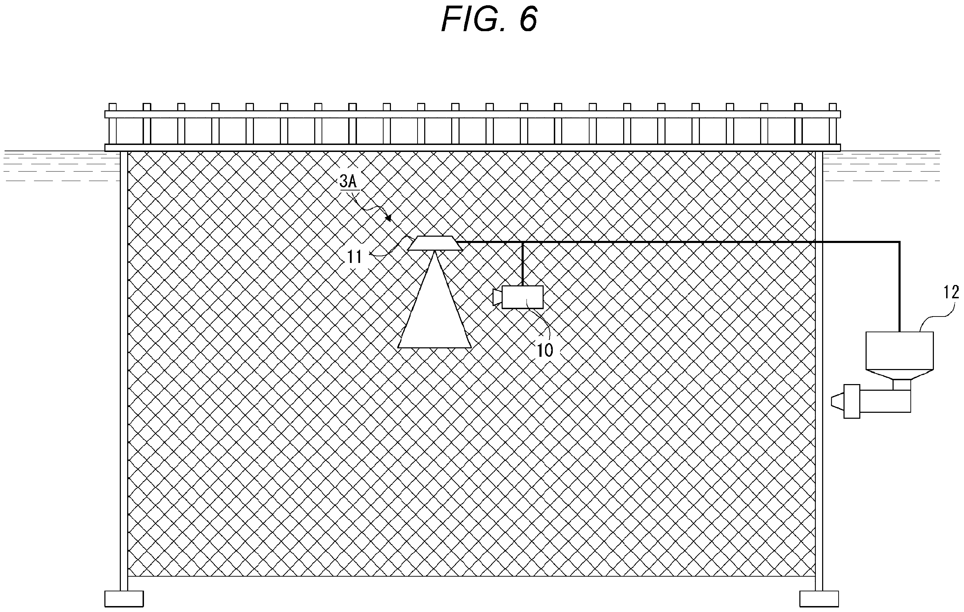

Fig. 6 illustrates an example of the purpose-specific operation. Here, the measurement device 3A that detects harmful plankton as the target object will be described as an example. The measurement device 3A includes not only the measurement unit 10 and the stimulus generation unit 11 but also a chemical spray unit 12.

The chemical spray unit 12 stores a chemical for exterminating harmful plankton. In the above purpose-specific measurement operation processing, in a case where harmful plankton serving as the target object is detected in step S16, the operation control unit 34 drives the chemical spray unit 12 to perform a purpose-specific operation of spraying a chemical to the cage in step S17. As described above, in the measurement device 3A, in a case where the purpose-specific measurement operation processing is performed, and harmful plankton serving as the target object is detected, a chemical for exterminating the harmful plankton is sprayed from the chemical spray unit 12.

Therefore, the measurement device 3A can exterminate harmful plankton in a case where the harmful plankton is detected.

Note that, although the measurement device 3A has been described as an example here, each of the measurement devices 3B and 3C also performs a purpose-specific operation set in advance in a case where a target object set in advance for each measurement device is detected. However, the purpose-specific operation performed in a case where the target object is detected may not only actually perform some operation like the measurement device 3A but also perform a software operation such as counting the number of operations.

### <3. Server Device>

### [3-1. Configuration of Server Device]

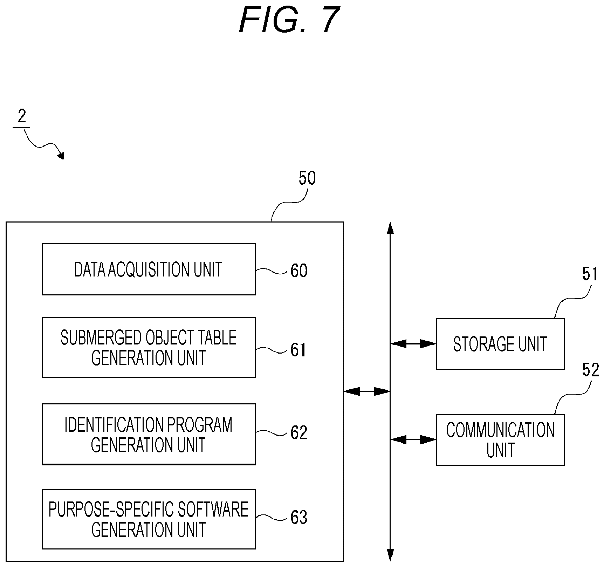

Fig. 7 illustrates a configuration of the server device 2. As illustrated in Fig. 7, the server device 2 includes a control unit 50, a storage unit 51, and a communication unit 52.

The control unit 50 includes, for example, a microcomputer including a CPU, a ROM, and a RAM and performs overall control of the server device 2. The control unit 50 functions as a data acquisition unit 60, a submerged object table generation unit 61, an identification program generation unit 62, and a purpose-specific software generation unit 63. Note that the data acquisition unit 60, the submerged object table generation unit 61, the identification program generation unit 62, and the purpose-specific software generation unit 63 will be described later in detail.

The storage unit 51 includes a nonvolatile memory. The communication unit 22 performs wired or wireless data communication with the measurement device 3.

The data acquisition unit 60 acquires (receives) new submerged object information (identification result) transmitted from the measurement device 3 and stores the new submerged object information in the storage unit 51 (medium). Here, the data acquisition unit 60 acquires new submerged object information transmitted from any of the measurement devices 3A, 3B, and 3C without distinction.

The submerged object table generation unit 61 generates a submerged object table (database) on the basis of the new submerged object information acquired by the data acquisition unit 60 and stores the generated submerged object table in the storage unit 51. Note that the generation of the submerged object table includes update (addition) of the submerged object table.

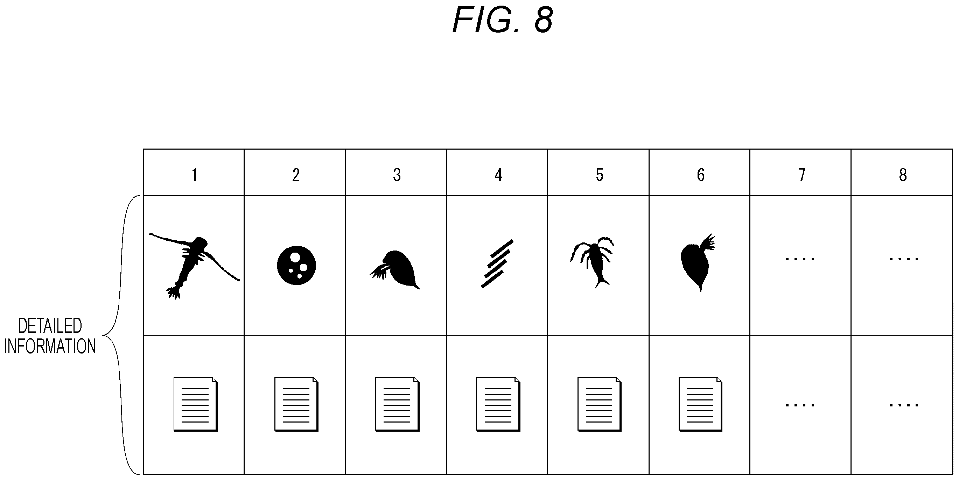

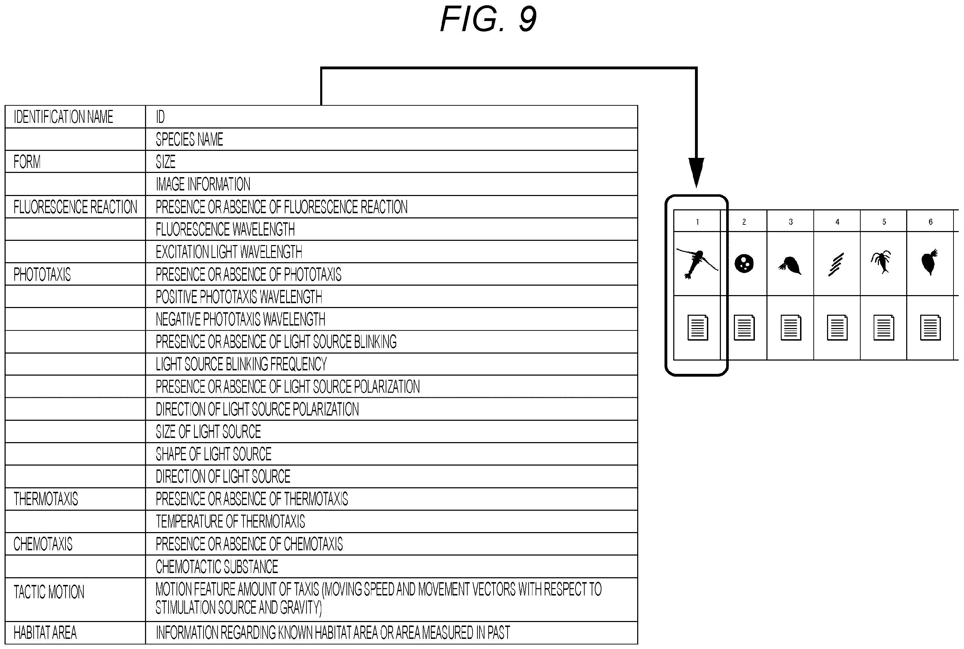

Fig. 8 shows the submerged object table. Fig. 9 shows detailed information of the submerged object table. As shown in Fig. 8, the submerged object table stores detailed information of each submerged object. As shown in Fig. 9, the detailed information includes information regarding an identification name, a form, a fluorescence reaction, a phototaxis, a thermotaxis, a chemotaxis, a tactic motion, and a habitat area.

For example, the identification name includes information regarding a unique ID and a species name. The form includes a size and image information. The fluorescence reaction includes information regarding the presence or absence of a fluorescence reaction, a fluorescence wavelength, and an excitation light wavelength. The phototaxis includes information regarding the presence or absence of the phototaxis, a positive phototaxis wavelength, a negative phototaxis wavelength, the presence or absence of light source blinking, a light source blinking frequency, the presence or absence of light source polarization, a direction of the light source polarization, a size of the light source, a shape of the light source, and a direction of the light source. The thermotaxis includes information regarding the presence or absence of the thermotaxis and a temperature of the thermotaxis. The chemotaxis includes information regarding the presence or absence of the chemotaxis and a chemotactic substance. The tactic motion includes information regarding a motion feature amount of the taxis (moving speed and movement vectors with respect to stimulation source and gravity). The habitat area includes information regarding a known habitat area or a measured area.

In a case where the new submerged object information is acquired by the data acquisition unit 60, the submerged object table generation unit 61 generates a submerged object table on the basis of the new submerged object information and stores the submerged object table in the storage unit 51.

Here, for example, the submerged object table generation unit 61 may specify a submerged object indicated by the new submerged object information by matching the new submerged object information with information regarding a submerged object indicated by an existing external database and add detailed information regarding the specified submerged object to the submerged object table.

Further, a user may analyze the new submerged object information and manually add the detailed information to the submerged object table.

Note that the new submerged object information and the information regarding the submerged object indicated by the existing external database may include only a part of each item of the above detailed information. In such a case, detailed information is added by combining (integrating) all or part of the matched information, and thus highly accurate detailed information can be registered.

Further, the submerged object information that is determined as a known submerged object by the measurement device 3 may be transmitted to the server device 2, and, based on the submerged object information, detailed information regarding the corresponding submerged object in the submerged object table may be further updated.

Therefore, the submerged object table generation unit 61 can extract information (first data portion) necessary for generating software to be used by the measurement device 3B from, for example, the new submerged object information (first data) acquired by the measurement device 3A, add all or part of the new submerged object information to the submerged object table such that the extracted information can be identified, and store the new submerged object information in the storage unit 51.

Further, the submerged object table generation unit 61 can integrate, for example, all or part of the new submerged object information acquired by the measurement device 3A and all or part of the new submerged object information (second data) acquired by the measurement device 3B, add the integrated information to the submerged object table as information necessary for generating the software to be used by the measurement device 3B, and store the information in the storage unit 51.

When the submerged object table is generated, the identification program generation unit 62 generates an identification program by machine learning using the generated submerged object table as training data. The identification program is a program for deriving the confidence rate between the submerged object detected by the measurement device 3 and the submerged object indicated by the submerged object table.

When generating the identification program, the identification program generation unit 62 transmits the same identification program to all the measurement devices 3 (3A, 3B, 3C). That is, the measurement device 3 identifies a submerged object by using the same identification program, regardless of the purpose.

Note that the identification program generation unit 62 may generate the identification program at predetermined intervals.

The purpose-specific software generation unit 63 generates software for each purpose of the measurement device 3. For example, the purpose-specific software generation unit 63 generates software in which a target object (target plankton) to be a target, a second threshold for identifying the target object, an operation performed when the target object is detected (purpose-specific operation), and the like are defined for each purpose. Note that, in the purpose-specific software, at least part of the target object is different, or the operation performed when the target object is detected is different.

Then, when generating software for each purpose, the purpose-specific software generation unit 63 transmits the generated software to the corresponding measurement device 3. In other words, the purpose-specific software generation unit 63 stores the purpose-specific software in the memory 21 (medium) of the measurement device 3 and thus generates the measurement device 3 storing the software.

For example, when acquiring the new submerged object information (first data) acquired by the measurement device 3A that executes the software for aquaculture water quality measurement serving as the first purpose, the server device 2 generates (updates) a submerged object table and an identification program on the basis of the new submerged object information and generates the software for deep-sea biological research serving as the second purpose on the basis of the submerged object table and the identification program. That is, when acquiring the new submerged object information acquired for the first purpose, the server device 2 generates the software for deep-sea biological research serving as the second purpose on the basis of the new submerged object information.

Further, when acquiring the new submerged object information (second data) acquired by the measurement device 3B that executes the software for deep-sea biological research serving as the second purpose, the server device 2 generates (updates) the submerged object table and the identification program on the basis of the new submerged object information and generates the software for aquaculture water quality measurement serving as the first purpose on the basis of the submerged object table and the identification program. That is, when acquiring the new submerged object information (second data) acquired for the second purpose, the server device 2 generates the software for deep-sea biological research serving as the first purpose on the basis of the new submerged object information.

Further, when acquiring the new submerged object information (first data) acquired by the measurement device 3A that executes the software for aquaculture water quality measurement serving as the first purpose, the server device 2 generates (updates) the submerged object table and the identification program on the basis of the new submerged object information and generates the software for aquaculture water quality measurement serving as the first purpose on the basis of the submerged object table and the identification program. That is, when acquiring the new submerged object information (first data) acquired for the first purpose, the server device 2 generates the software for aquaculture water quality measurement serving as the first purpose on the basis of the new submerged object information.

Further, when acquiring the new submerged object information (first data) acquired by the measurement device 3A that executes the software for aquaculture water quality measurement serving as the first purpose and the new submerged object information (second data) acquired by the measurement device 3B that executes the software for deep-sea biological research serving as the second purpose, the server device 2 generates (updates) the submerged object table and the identification program on the basis of the new submerged object information and generates the software for aquaculture water quality measurement serving as the first purpose and the software for deep-sea biological research serving as the second purpose on the basis of the submerged object table and the identification program. That is, when acquiring the new submerged object information (first data) acquired for the first purpose and the new submerged object information (second data) acquired for the second purpose, the server device 2 generates the software for aquaculture water quality measurement serving as the first purpose and the software for aquaculture water quality measurement serving as the second purpose on the basis of the new submerged object information.

As described above, the plurality of measurement devices 3 (3A, 3B, 3C) is networked and connected to the server device 2 in the underwater measurement system 1, and thus the server device 2 can perform learning on the basis of submerged object information measured at different measurement places in a wide range and can efficiently generate various pieces of purpose-specific software.

### [3-2. Generation Processing]

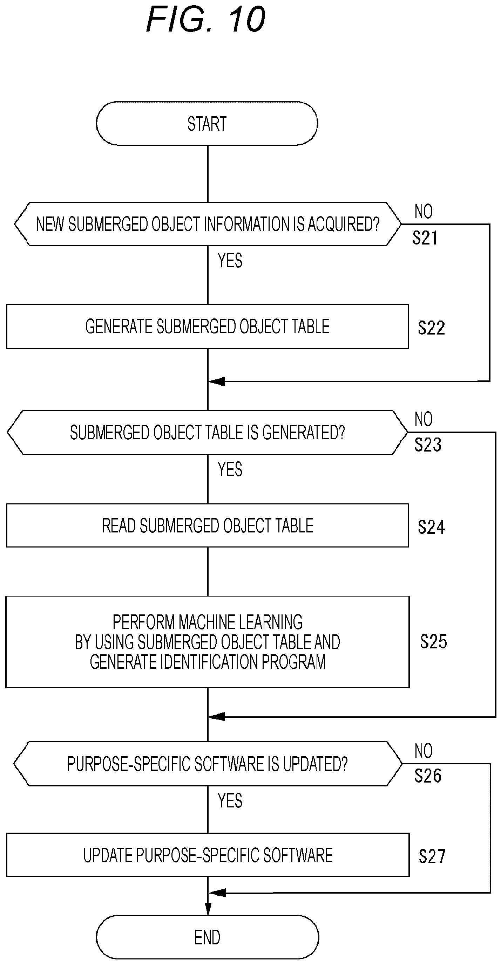

Fig. 10 is a flowchart showing a procedure of generation processing. As shown in Fig. 10, in step S21, the submerged object table generation unit 61 determines whether or not new submerged object information has been acquired by the data acquisition unit 60. As a result, in a case where it is determined that the new submerged object information has been acquired (Yes in step S21), in step S22, the submerged object table generation unit 61 reads the submerged object table stored in the storage unit 51 and generates (adds) the submerged object table on the basis of the new submerged object information acquired by the data acquisition unit 60.

In step S23, the identification program generation unit 62 determines whether or not the submerged object table has been generated. As a result, in a case where it is determined that the submerged object table has been generated (Yes in step S23), the identification program generation unit 62 reads the generated submerged object table in step S24. Then, in step S25, the identification program generation unit 62 performs machine learning by using the submerged object table and generates an identification program.

In step S26, the purpose-specific software generation unit 63 determines whether to update the software. Here, for example, in a case where the identification program is updated, in a case where an operation for updating the identification program is performed by the user, or in other cases, it is determined that the software is to be updated. Then, in a case where the software is updated (Yes in step S26), the purpose-specific software generation unit 63 updates the target software that needs to be updated and transmits the updated software to the measurement device 3.

### <4. Another Configuration Example of Underwater Measurement System>

Note that the embodiments are not limited to the specific examples described above and may be configured as various modification examples.

The measurement device 3 of the above embodiment is an example and may have another configuration as long as the measurement device can measure at least a submerged object.

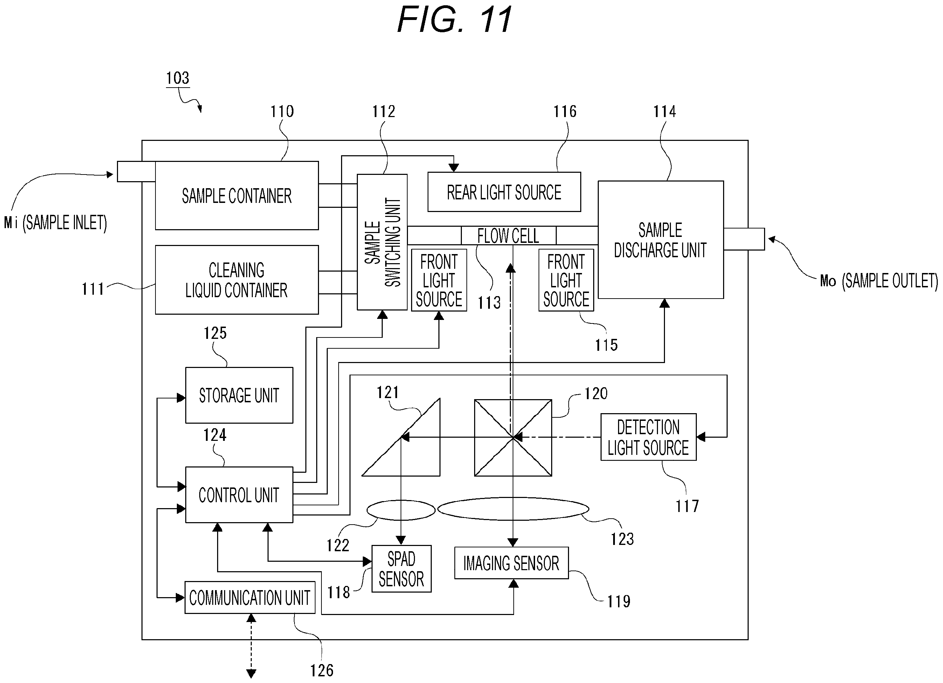

Fig. 11 illustrates a configuration of a measurement device 103 according to a modification example. As illustrated in Fig. 11, the measurement device 103 includes a sample container 110, a cleaning liquid container 111, a sample switching unit 112, a flow cell 113, a sample discharge unit 114, a front light source 115, a rear light source 116, a detection light source 117, a single photon avalanche diode (SPAD) sensor 118, an imaging sensor 119, a one-way mirror 120, a mirror 121, a lens 122, a lens 123, a control unit 124, a storage unit 125, and a communication unit 126.

The sample container 110 is a container that stores fluid (sea water or lake water) as a sample and stores a sample taken in from the outside of the device via a sample inlet Mi.

The cleaning liquid container 111 is a container that stores a cleaning liquid for cleaning a flow channel in the flow cell 113.

The sample switching unit 112 switches fluid caused to flow into the flow channel in the flow cell 113 between the sample from the sample container 110 and the cleaning liquid from the cleaning liquid container 111.

The flow cell 113 functions as a sample containing unit, and the fluid serving as the sample is sampled from the flow channel formed thereinside. Note that, as described later, the cleaning liquid flows into the flow channel of the flow cell 113 in a state in which the sample switching unit 112 is switched to the cleaning liquid container 111.

The sample discharge unit 114 has a pump for discharging fluid and drives the pump to discharge the fluid in the flow channel of the flow cell 113 via a sample outlet Mo located outside the device.

Here, in the present example, a flow channel from the sample container 110 to the sample discharge unit 114 via the sample switching unit 112 and the flow cell 113 and a flow channel from the cleaning liquid container 111 to the sample discharge unit 114 via the sample switching unit 112 and the flow cell 113 are provided, and the pump of the sample discharge unit 114 is driven to cause the sample to flow into the flow cell 113 from the sample container 110 and to cause the cleaning liquid to flow into the flow cell 113 from the cleaning liquid container 111.

The front light source 115 is a light source for irradiating the fluid in the flow cell 113 when imaging is performed by the imaging sensor 119. The "front" here means a surface on the imaging sensor 119 side with respect to a position of the flow cell 113. In the modification example, the front light source 115 is an annular light source, is prevented from hindering imaging by the imaging sensor 119, and irradiates the sample obliquely from the front side of the flow cell 113.

The rear light source 116, as well as the front light source 115, is a light source for irradiating the fluid in the flow cell 113 when imaging is performed by the imaging sensor 119 and is located on the side opposite to the front light source 115 with respect to the flow cell 113.

The rear light source 116 is used for bright-field imaging. The imaging sensor 119 receives light transmitted through the sample, which is similar to a method used in a general microscope. Because illumination light is directly incident on the lens 15, the background is bright.

Meanwhile, the front light source 115 is used for dark-field imaging. A sample is irradiated with light from an oblique side of the sample, and the imaging sensor 119 receives scattered light and reflected light of the target object. Even a transparent target object can be measured finely with high contrast. In this case, because the illumination light is not directly incident on the lens 15, the background is dark.

The detection light source 117 emits light for detecting a target object toward the sample sampled by the flow cell 113. The detection light source 117 is, for example, a semiconductor laser or the like. As illustrated in Fig. 11, light emitted from the detection light source 117 is reflected by the one-way mirror 120 to irradiate the fluid sampled in the flow channel in the flow cell 113.

The SPAD sensor 118 functions as a sensor for detecting a submerged object in the fluid in the flow cell 113. In order to detect weak light (excited light or the like) of microorganisms or particles, the measurement device 103 includes a pixel array in which a plurality of photodetection pixels is arrayed. One technique of this photodetection pixel is, for example, SPAD. In the SPAD, avalanche amplification occurs when one photon enters a PN junction region of a high electric field in a state in which a voltage larger than a breakdown voltage is applied. At that time, a position and timing of a pixel through which a current instantaneously flows are detected, and thus it is possible to specify the presence or absence, position, size, and the like of the submerged object in the flow cell 113.

The SPAD sensor 118 includes a SPAD element that performs photoelectric conversion on incident light by using an electron avalanche phenomenon. The electron avalanche phenomenon in the SPAD element is a kind of phenomenon known as an internal photoelectric effect. The internal photoelectric effect is a phenomenon in which conduction electrons in a substance increase when a semiconductor or an insulator is irradiated with light.

As is known, the SPAD element is an element having a light receiving resolution in units of photons. In other words, the element can identify whether or not light is received in units of photons.

The SPAD sensor 118 in the present example has a configuration in which a plurality of pixels including the SPAD elements is two-dimensionally arrayed.

Light emitted from the submerged object in the fluid in the flow cell 113 is incident on the SPAD sensor 118 via the one-way mirror 120, the mirror 13, and the lens 14.

The imaging sensor 119 is configured as, for example, a CCD, CMOS, or other types of image sensor, and a plurality of pixels including photoelectric conversion elements is two-dimensionally arrayed. The photoelectric conversion element included in each pixel of the imaging sensor 119 does not perform photoelectric conversion by using the electron avalanche phenomenon, and, for example, a photoelectric conversion element used in general imaging, such as a photodiode, is adopted. That is, the photoelectric conversion element has a lower light receiving resolution than the SPAD element.

The imaging sensor 119 captures an image of the flow channel in the flow cell 113 (an image of at least the flow channel in an imaging field of view). Light (image light) from the flow cell 113 is transmitted through the one-way mirror 120 and is incident on the imaging sensor 119 via the lens 15.

The control unit 124 includes, for example, a microcomputer including a CPU, a ROM, and a RAM and performs overall control of the measurement device 103. For example, the control unit 124 performs switching control of the sample switching unit 112, light emission drive control of the front light source 115 and the rear light source 116, drive control of the pump in the sample discharge unit 114, light emission drive control of the detection light source 117, and the like.

Further, the control unit 124 performs processing of reading data stored in the storage unit 125, processing of storing data in the storage unit 125, and exchange of various kinds of data with an external device via the communication unit 126. For example, the storage unit 125 includes a nonvolatile memory. The communication unit 126 performs wired or wireless data communication with the external device.

Further, the control unit 124 of the present example performs submerged object detection processing based on a light reception signal by the SPAD sensor 118, various kinds of image analysis processing based on an image captured by the imaging sensor 119, and the like.

Further, the submerged object is measured by using the taxis and fluorescence of the submerged object in the embodiment and modification example described above, but the submerged object may be measured by a different method.

Further, in the embodiment and modification example described above, a case where the submerged object table is stored in the storage unit 51 of the server device 2. However, the submerged object table may be stored in the memory 21 of the measurement device 3.

### <5. Summary>

As described above, the server device 2 according to the embodiment includes: the data acquisition unit 60 that receives first data regarding a submerged object acquired for a first purpose; and the purpose-specific software generation unit 63 that generates software to be used for a second purpose different from the first purpose on the basis of the first data.

Therefore, the software to be used for the second purpose can be generated on the basis of the first data acquired for the first purpose different from the second purpose.

Therefore, software to be used for different purposes can be efficiently generated.

In the server device 2 according to the above present technology, the purpose-specific software generation unit 63 may generate software to be used for the first purpose on the basis of second data regarding a submerged object acquired for the second purpose.

Therefore, the software to be used for the first purpose can be generated on the basis of both the first data regarding the submerged object acquired for the first purpose and the second data acquired for the second purpose different from the first purpose.

Therefore, both the first data and the second data are used to generate the software to be used for the first purpose, and thus the software to be used for the first purpose can be more efficiently generated.

In the server device 2 according to the above present technology, the purpose-specific software generation unit 63 may generate software to be used for the first purpose on the basis of the first data.

Therefore, both the software to be used for the first purpose and the software to be used for the second purpose can be generated on the basis of the first data acquired for the first purpose.

Therefore, the software to be used for the first purpose and the software to be used for the second purpose can be more efficiently generated on the basis of the first data acquired for one purpose.

In the server device 2 according to the above present technology, the purpose-specific software generation unit 63 may generate software to be used for the first purpose and the software to be used for the second purpose on the basis of the first data and second data regarding a submerged object acquired for the second purpose.

Therefore, both the software to be used for the first purpose and the software to be used for the second purpose can be generated on the basis of both the first data acquired for the first purpose and the second data acquired for the second purpose.

Therefore, the software to be used for the first purpose and the software to be used for the second purpose can be efficiently generated on the basis of the first data and the second data acquired for different purposes.

The server device 2 according to the above present technology may further include an identification program generation unit 62 that generates an identification program for identifying the submerged object in software to be used for the first purpose and the software to be used for the second purpose.

Therefore, it is possible to identify the submerged object by using the identification program in the software to be used for the first purpose and the software to be used for the second purpose.

Therefore, the submerged object can be identified on the basis of the identification program.

In the server device 2 according to the above present technology, the identification program generation unit 62 may be used in common to the software to be used for the first purpose and the software to be used for the second purpose.

Therefore, it is possible to identify the submerged object by using the common identification program in the software to be used for the first purpose and the software to be used for the second purpose.

Therefore, it is unnecessary to separately generate the identification program, and the software can be efficiently generated.

In the server device 2 according to the above present technology, a database used to generate the identification program may be generated on the basis of the first data and second data regarding a submerged object acquired for the second purpose.

Therefore, the database is generated on the basis of both the first data regarding the submerged object acquired for the first purpose and the second data regarding the submerged object acquired for the second purpose.

Therefore, the database can be efficiently generated on the basis of the first data and the second data.

In the server device 2 according to the above present technology, at least part of a submerged object to be identified may be different between the first purpose and the second purpose.

Therefore, the software to be used for the second purpose can be generated on the basis of the first data acquired for the first purpose in which at least part of the submerged object to be identified is different.

Therefore, even if the target submerged object is different for each purpose, purpose-specific software can be efficiently generated.

In the server device 2 according to the above present technology, an operation performed when the submerged object to be identified is detected may be different between the first purpose and the second purpose.

Therefore, the software to be used for the second purpose can be generated on the basis of the first data acquired for the first purpose in which the operation performed when the submerged object to be identified is detected is different.

Therefore, even if operations to be performed are different, the purpose-specific software can be efficiently generated.

An electronic device (measurement device 3) generation method according to the present technology includes: receiving first data regarding a submerged object acquired for a first purpose; generating software to be used for a second purpose different from the first purpose on the basis of the first data; and storing the software in a medium (memory 21).

The electronic device (measurement device 3) generation method according to the above present technology may further include: acquiring second data regarding a submerged object acquired for the second purpose; acquiring an identification result obtained by processing the second data by using the software to be used for the second purpose; and storing the identification result in the medium.

Such the electronic device generation method can also obtain effects similar to those of the server device 2 according to the above present technology.

A database (submerged object table) generation method according to the present technology includes: acquiring first data regarding a submerged object acquired for a first purpose; extracting, from the first data, a first data portion necessary for generating software to be used for a second purpose different from the first purpose; and storing all or part of the first data in a medium such that the first data portion is identifiable.

The database (submerged object table) generation method according to the above present technology may further include: acquiring second data regarding a submerged object acquired for the second purpose; and integrating the second data and the first data portion and storing the integrated second data and first data portion in a medium as information necessary for generating the software to be used for the second purpose.

Such the database generation method can also obtain effects similar to those of the server device 2 according to the above present technology.

An electronic device (measurement device 3) according to the present technology includes a medium storing the database.

Such the electronic device can also obtain effects similar to those of the server device 2 according to the above present technology.

Note that the effects described in the present specification are merely illustrative and are not limited. Further, additional effects may be obtained.

### <6. Present Technology>

The present technology can also be configured as follows.
(1) A server device including:
   a data acquisition unit that receives first data regarding a submerged object acquired for a first purpose; and
   a purpose-specific software generation unit that generates software to be used for a second purpose different from the first purpose on the basis of the first data.
(2) The server device according to (1), in which
   the purpose-specific software generation unit generates software to be used for the first purpose on the basis of second data regarding a submerged object acquired for the second purpose.
(3) The server device according to (1) or (2), in which
   the purpose-specific software generation unit generates software to be used for the first purpose on the basis of the first data.
(4) The server device according to any one of (1) to (3), in which
   the purpose-specific software generation unit generates software to be used for the first purpose and the software to be used for the second purpose on the basis of the first data and second data regarding a submerged object acquired for the second purpose.
(5) The server device according to any one of (1) to (4), further including
   an identification program generation unit that generates an identification program for identifying the submerged object in software to be used for the first purpose and the software to be used for the second purpose.
(6) The server device according to (5), in which
   the identification program is used in common to the software to be used for the first purpose and the software to be used for the second purpose.
(7) The server device according to (5) or (6), in which
   a database used to generate the identification program is generated on the basis of the first data and second data regarding a submerged object acquired for the second purpose.
(8) The server device according to any one of (1) to (7), in which
   at least part of a submerged object to be identified is different between the first purpose and the second purpose.
(9) The server device according to any one of (1) to (8), in which
   an operation performed when the submerged object to be identified is detected is different between the first purpose and the second purpose.
(10) A generation method including:
   receiving first data regarding a submerged object acquired for a first purpose; and
   generating software to be used for a second purpose different from the first purpose on the basis of the first data.
(11) An electronic device generation method including:
   receiving first data regarding a submerged object acquired for a first purpose;
   generating software to be used for a second purpose different from the first purpose on the basis of the first data; and
   storing the software in a medium.
(12) The electronic device generation method according to (11), further including:
   acquiring second data regarding a submerged object acquired for the second purpose;
   acquiring an identification result obtained by processing the second data by using the software to be used for the second purpose; and
   storing the identification result in the medium.
(13) A database generation method including:
   acquiring first data regarding a submerged object acquired for a first purpose;
   extracting, from the first data, a first data portion necessary for generating software to be used for a second purpose different from the first purpose; and
   storing all or part of the first data in a medium such that the first data portion is identifiable.
(14) The database generation method according to (13), further including:
   acquiring second data regarding a submerged object acquired for the second purpose; and
   integrating the second data and the first data portion and storing the integrated second data and first data portion in a medium as information necessary for generating the software to be used for the second purpose.
(15) An electronic device including
   a medium storing the database according to (13) or (14).
(16) An electronic device including:
   a communication unit that acquires software to be used for a second purpose different from a first purpose, the software being generated on the basis of first data regarding a submerged object acquired for the first purpose; and
   a medium storing the acquired software.
(17) An information processing system including a server device and an electronic device, in which:
   the server device includes
   a data acquisition unit that acquires first data regarding a submerged object acquired for a first purpose,
   a purpose-specific software generation unit that generates software to be used for a second purpose different from the first purpose on the basis of the first data, and
   a communication unit that transmits the software; and
   the electronic device includes
   a communication unit that acquires the software, and
   a medium storing the acquired software.
(18) A program for causing a computer to function as:
   a data acquisition unit that acquires first data regarding a submerged object acquired for a first purpose; and
   a purpose-specific software generation unit that generates software to be used for a second purpose different from the first purpose on the basis of the first data.

### REFERENCE SIGNS LIST

- 1: Underwater measurement system
- 2: Server device
- 3: Measurement device (electronic device)
- 60: Data acquisition unit
- 61: Submerged object table generation unit
- 62: Identification program generation unit
- 63: Purpose-specific software generation unit

## Claims

1. A server device comprising:
a data acquisition unit that acquires first data regarding a submerged object acquired for a first purpose; and
a purpose-specific software generation unit that generates software to be used for a second purpose different from the first purpose on a basis of the first data.

2. The server device according to claim 1, wherein
the purpose-specific software generation unit generates software to be used for the first purpose on a basis of second data regarding a submerged object acquired for the second purpose.

3. The server device according to claim 1, wherein
the purpose-specific software generation unit generates software to be used for the first purpose on a basis of the first data.

4. The server device according to claim 1, wherein
the purpose-specific software generation unit generates software to be used for the first purpose and the software to be used for the second purpose on a basis of the first data and second data regarding a submerged object acquired for the second purpose.

5. The server device according to claim 1, further comprising
an identification program generation unit that generates an identification program for identifying the submerged object in software to be used for the first purpose and the software to be used for the second purpose.

6. The server device according to claim 5, wherein
the identification program is used in common to the software to be used for the first purpose and the software to be used for the second purpose.

7. The server device according to claim 5, wherein
a database used to generate the identification program is generated on a basis of the first data and second data regarding a submerged object acquired for the second purpose.

8. The server device according to claim 1, wherein
at least part of a target submerged object is different between the first purpose and the second purpose.

9. The server device according to claim 1, wherein
an operation performed when the target submerged object is detected is different between the first purpose and the second purpose.

10. A generation method comprising:
acquiring first data regarding a submerged object acquired for a first purpose; and
generating software to be used for a second purpose different from the first purpose on a basis of the first data.

11. An electronic device generation method comprising:
acquiring first data regarding a submerged object acquired for a first purpose;
generating software to be used for a second purpose different from the first purpose on a basis of the first data; and
storing the software in a medium.

12. The electronic device generation method according to claim 11, further comprising:
acquiring second data regarding a submerged object acquired for the second purpose;
acquiring an identification result obtained by processing the second data by using the software to be used for the second purpose; and
storing the identification result in the medium.

13. A database generation method comprising:
acquiring first data regarding a submerged object acquired for a first purpose;
extracting, from the first data, a first data portion necessary for generating software to be used for a second purpose different from the first purpose; and
storing all or part of the first data in a medium such that the first data portion is identifiable.

14. The database generation method according to claim 13, further comprising:
acquiring second data regarding a submerged object acquired for the second purpose; and
integrating the second data and the first data portion and storing the integrated second data and first data portion in a medium as information necessary for generating the software to be used for the second purpose.

15. An electronic device comprising
a medium storing the database according to claim 13.
